# EUROPEAN PATENT APPLICATION

(11) **EP 1 906 185 A1**
(43) Date of publication of application: **02.04.2008**
(21) Application number: 07006379.7
(22) Date of filing: 28.03.2007
(51) Int. Cl.: G01N 33/574, C07K 14/72

(54) **Use of at least one isoform of progesterone receptor membrane component 1 (PGRMC1)**

(30) Priority: 26.09.2006 WO PCT/EP2006/009351
(71) Applicant: ProteoSys AG, 55129 Mainz (DE)
(72) Inventor: Cahill, Michael, 55296 Loerzweiler (DE); Neubauer, Hans, 72074 Tübingen (DE)
(74) Representative: Patentanwälte Ruff, Wilhelm, Beier, Dauster & Partner

(57) **Abstract**

The invention relates to the use of at least one isoform of progesterone receptor membrane component 1 (PGRMC1) as diagnostic marker and/or therapeutic target for diseases associated with neogenin and/or DCC and/or for the manufacture of a medicament for treatment of diseases associated with neogenin and/or DCC, the use of at least one reagent that influences the function of at least one isoform of PGRMC1 for the manufacture of a medicament for treatment of diseases associated with neogenin and/or DCC, the use of at least one isoform of PGRMC1 for influencing abundance or subcellular localisation of at least one class of molecules interacting with PGRMC1, an assay kit for diagnosis and/or therapy of diseases associated with neogenin and/or DCC, comprising at least one isoform of PGRMC1 and/or at least one reagent that influences the function of at least one isoform of PGRMC1, the use of at least one isoform of PGRMC1 as diagnostic marker in diagnosis for diseases associated with aberrant biological phenotypes, wherein the phosphorylation status of the at least one isoform of PGRMC1 is determinedland or estimated and the use of at least one reagent for influencing the abundance and/or activity of isoforms of proteins for diagnosis and/or therapy of diseases associated with aberrant biological phenotypes, wherein said proteins are involved in protein interaction or multi-protein complexes with either at least one isoform of phosphorylated or non-phosphorylated PGRMC1.

## Description

The invention relates to the use of at least one isoform of progesterone receptor membrane component 1 (PGRMC1) as diagnostic marker and/or therapeutic target, the use of at least one isoform of progesterone receptor membrane component 1 (PGRMC1) for the manufacture of a medicament, the use of at least one reagent that influences the function of at least one isoform of progesterone receptor membrane component 1 (PGRMC1) for the manufacture of a medicament, the use of at least one isoform of progesterone receptor membrane component 1 (PGRMC1) for influencing at least one class of molecules interacting with PGRMC1, to assay kits for diagnosis and/or therapy of diseases, to the use of at least one isoform of progesterone receptor membrane component 1 (PGRMC1) as diagnostic marker in diagnosis for diseases associated with aberrant biological phenotypes and to the use of at least one reagent for influencing, in particular increasing or inhibiting, the abundance and/or activity of isoforms of proteins for diagnosis and/or therapy of diseases associated with aberrant biological phenotypes.

Breast cancer is one of the most common forms of cancer observed in women, with more than 180,000 new cases and over 40,000 deaths expected in the USA in 2007 [1]. Endogenous estrogens, which have effects on many organs, are thought to play a major role in the development of the breast, suggesting that an increased sensitivity or longer exposures to estrogens is involved in a higher risk of tumorigenesis [2-4].

The classical estrogen receptor (ERα: hereafter ER) is found in 50-80% of breast tumors and ER status is essential in making decisions about endocrine therapy with anti-estrogens which inhibit the mitogenic activity of estrogens in breast cancer. There are three classes of anti-estrogens currently in clinical use: selective estrogen receptor modulators (SERMs, e.g., tamoxifen), aromatase inhibitors and, "pure" estrogen antagonists such as fulvestrant which, like tamoxifen, binds to estrogen receptors competitively. However, in contrast to tamoxifen, fulvestrant's binding leads to rapid degradation and loss of the ER protein [5;6].

Clinically, a positive ER status correlates with favorable prognostic features, including a lower rate of cell proliferation and histological evidence of tumor differentiation. ER status is also prognostic for the site of gross metastatic spread. For reasons unknown, ER+ tumors are more likely to initially manifest clinically apparent metastases in bone, soft tissue, or the reproductive and genital tracts, whereas ER- tumors more commonly metastasize to brain and liver. Several studies have correlated ER expression to lower Matrigel invasiveness and reduced metastatic potential of breast cancer cell lines [7;8].

Moreover, when ER+ cells are implanted in nude mice, tumors appear only in the presence of estrogens and are poorly metastatic as compared to those developed from ER- breast cancer cell lines [9; 1 0]. This paradox suggests that ER expression could be associated with or involved in pathways that hinder cancer progression.

At the mRNA level, gene expression analysis has revealed that different molecular subtypes of tumour exist within the broader groups of ER+ and ER- breast cancers, and these are associated with different clinical outcomes. ER+ tumors exist in at least two subtypes, luminal A and luminal B, which vary markedly in gene expression and prognosis [11]. Conversely, hormone-receptor-negative breast cancer comprises two distinct subtypes, the Her2 subtype and the basal-like subtype [11;12], which differ in biology and behaviour. Both of these are associated with a worse clinical outcome than ER+ tumors.

Importantly a very similar subdivision of breast cancers has been produced using immunohistochemistry to analyze patterns of protein expression in tumor sections and, which suggests that a few protein biomarkers can be used to stratify breast cancers into different groups [13;14]. One set of biomarkers is built by the family of cytokeratins (CKs). They can be grouped into the "luminal CKs" 7/8, 18, and 19 and into the "basal CKs" 5/6 and 14 [13].

In addition to these molecular portraits, it has been shown that expression patterns present in primary breast cancers are also observed in their respective metastases [15]. Other gene expression profiles have distinguished breast cancers according to the differential expression of a wound response signature. Twenty years ago, based on the observation that there are many histological similarities between the tumor microenvironment and normal wound healing, it was proposed by Dvorak that the tumor stroma is "normal wound heating gone awry" [16]. Since then it has been discovered that genes induced in a fibroblast serum-response program are expressed in tumors by the tumor cells themselves, by tumor-associated fibroblasts, or both [17]. The molecular features that define this wound-like phenotype are evident at an early clinical stage, persist during treatment, arid predict increased risk of metastasis and death in breast, lung, and gastric carcinomas.

The properties and biology of the PGRMC1 protein have been recently reviewed [18]. Briefly, progesterone binding has only been observed in biological fractions containing the protein PAIRBP1 in addition to PGRMC1. Various other steroid and cholesterol-related ligands could potentially affect PGRMC1 biology. A biological role of heme binding has been implicated, and PGRMC1 has been shown to be present in protein complexes with various cytochrome P450 enzymes. Various target peptides for interaction with proteins involved with signal transduction are present in PGRMC1, and the phosphorylation of various amino acids that would regulate such interactions have been observed in living cells. PGRMC1 also contains evolutionarily conserved motifs that are present in the protein structure at positions which could indicate a function in endocytosis or other membrane trafficking events (reviewed in reference [18]).

Other homologues of PGRMC1 are known for example from Ceanorhabdidtis elegans. The Vem-1 *Ceanorhabdidtis elegans* homologue of PGRMC1 interacts genetically and physically with the Unc-40 transmembrane receptor that is involved in axonal guidance [19]. Unc-40 is the receptor for Unc-6, a lamin-like molecule that acts as neuronal attraction signal and is homologous to the mammalian Netrin family [25]. Unc-40 has two mammalian homologues; Deleted in Colorectal Cancer (DCC) and Neogenin. These immunoglobulin domain transmembrane proteins act as receptors for proteins of the Netrin family, which also bind receptors of the mammalian Unc-5 family, in a system of axon guidance whose underlying components are conserved between nematodes and mammals [26-30]. The repulsive guidance molecule (RGM) family are membrane-bound molecules which 'also signal through the Neogenin receptor [31], as well as other receptors in axon guidance [32]. Thus migrating axons are not only attracted towards sources of Netrins, but axons growing in other directions undergo programmed cell death due to the lack of Netrin. This apoptosis depends on the presence of DCC or Neogenin [33].

Furthermore, in WO2006029836 differentially abundant isoforms of PGRMC1 in breast cancers that differed in expression of the estrogen receptor (ER) were identified. It was also demonstrated that these isoforms differ in their phosphorylation status, and that the presence of certain phosphorylation sites of PGRMC1 affected cell survival in stable cloning experiments (PCT/EP2006/009351).

Surprisingly it has now been found that PGRMC1 is a central orchestrator of biological processes which determine the pathogenicity of not only cancer cells, but indeed occupies a regulatory crossroad function providing a nexus point for the regulation of higher order biological responses in multiple biological systems. As such PGRMC1 represents a suitable target molecule for pharmaceutical cancer treatment, especially of reagents which affect phosphorylation and/or intracellular localisation of PGRMC1. Furthermore, analogous applications exist in the fields of nervous system diseases, inflammatory diseases, and cardiovascular diseases.

### In the scope of the present the following terms shall be defined:

The term "phosphorylation status" as used herein comprises the absolute or relative degree of phosphorylation of proteins and/or reagents.
The term "aberrant biological phenotypes" as used herein comprises all forms of aberrant biological in vivo manifestations, for instance uncontrolled cell proliferation.

"Activity" of proteins as used herein, comprises the enzymatic activity, binding affinity and/or posttranslational activity, in particular phosphorylation.

The term "abundance" as used herein is equivalent to the expression level of proteins, in particular of mPR, being detectable with prior art methods.

The terms "neogenin" and "Deleted in colorectal cancer (DCC)" as used herein preferably shall include any family member of these receptors and/or neogenin-like and/or DCC-like receptors which can be influenced by PGRMC1.

The present invention is accordingly directed to the use of at least one isoform of progesterone receptor membrane component 1 (PGRMC1) as diagnostic marker and/or therapeutic target for diseases according to claim 1, the use of at least one isoform of progesterone receptor membrane component 1 (PGRMC1) for the manufacture of a medicament for treatment of diseases according to claim 2 and the use of at least one reagent that influences the function of at least one isoform of progesterone receptor membrane component 1 (PGRMC1) for the manufacture of a medicament for treatment of diseases according to claim 2. Preferred embodiments of use claims 1 to 3 are specified in dependent claims 4 to 13 and 19 to 21.

The present invention is further directed to the use of at least one isoform of progesterone receptor membrane component 1 (PGRMC1) for influencing abundance or subcellular localisation of at least one class of molecules interacting with PGRMC1 according to claims 14 and 15 and to Assay kits according to claims 22 and 23. Preferred embodiments of use claims 14 and 15 are specified in dependent claims 16 to 18.

The present invention is further directed to the use of at least one isoform of progesterone receptor membrane component 1 (PGRMC1) as diagnostic marker in diagnosis for diseases according to claim 24 and to the use of at least one reagent for influencing, in particular increasing or inhibiting, the abundance and/or activity of isoforms of proteins for diagnosis and/or therapy of diseases according to claim 26. Preferred embodiments of use claims 24 and 26 are specified in dependent claims 25 and 27.

The wording of all claims, including dependent claims, is hereby incorporated in the description by reference.

According to the present invention at least one isoform of progesterone receptor membrane component 1 (PGRMC1) is used
- as diagnostic marker and/or therapeutic target for diseases associated with neogenin and/or DCC and/or
- for the manufacture of a medicament for treatment of diseases associated with neogenin and/or DCC.

Further, according to the present invention at least one reagent that influences the function of at least one isoform of progesterone receptor membrane component 1 (PGRMC1) is used for the manufacture of a medicament for treatment of diseases associated with neogenin and/or DCC.

According to a preferred embodiment of the invention said at least one reagent increases or decreases the function or changes the subcellular locality of of PGRMC1.

According to a preferred embodiment of the invention said at least one reagent is a reagent for influencing, in particular increasing or decreasing, the phosphorylation status of the at least one isoform of PGRMC1.

According to a preferred embodiment of the invention said at least one reagent is an antibody and/or a small molecule affinity reagent directed against PGRMC1.

According to a preferred embodiment at least two or three isoforms are used as diagnostic marker and/or therapeutic target or in the manufacture of the medicament.

Further it is preferred that said diseases comprise neuronal disorders, preferably memory disorders, in particular in mammalians.

It may also be preferred that said diseases comprise cancer of the nervous system.

Said diseases further may comprise cancer, in particular breast cancer.

In a preferred embodiment said diseases comprise inflammatory diseases, in particular atherosclerosis or rheumatoid arthritis, and/or cardiovascular diseases.

According to a preferred embodiment of the invention at least one of said isoforms of PGRMC1 has a significantly increased abundance in cancer cells of said cancers.

According to a preferred embodiment of the invention said isoforms of PGRMC1 differ from each other in their phosphorylation status.

Further it is preferred that said cancer cells are negative for the estrogen receptor (ER-) and/or negative for the progesterone receptor (PR-).

According to the present invention at least one isoform of progesterone receptor membrane component 1 (PGRMC1) is used
- for influencing, in particular increasing, abundance or subcellular localisation of at least one class of molecules interacting with PGRMC1 and/or
- for influencing, in particular decreasing, abundance or subcellular localisation of at least one class of molecules interacting with PGRMC1.

According to a preferred embodiment of the invention said class of molecule is at least one of Deleted in colorectal cancer (DCC) or Neogenin.

According to a preferred embodiment of the invention said class of molecule is a ubiquitin ligase.

According to a preferred embodiment of the invention said class of molecule is a polyubiquitinase.

The invention is further directed to an Assay kit for diagnosis and/or therapy of diseases associated with neogenin and/or DCC, comprising at least one isoform of progesterone receptor membrane component 1 (PGRMC1) and to an Assay kit for diagnosis and/or therapy of diseases associated with neogenin and/or DCC, comprising at least one reagent that influences the function of at least one isoform of progesterone receptor membrane component 1 (PGRMC1).

The present invention is further directed to the use of at least one isoform of progesterone receptor membrane component 1 (PGRMC1) as diagnostic marker in diagnosis for diseases associated with aberrant biological phenotypes, wherein the phosphorylation status of the at least one isoform of progesterone receptor membrane component 1 (PGRMC1) is determined/and or estimated.

According to a preferred embodiment of the invention the phosphorylation status is determined and/or estimated by analysis of proteins that are involved, in particular differentially involved, in protein interaction or multi-protein complexes with either phosphorylated or non-phosphorylated at least one isoform of progesterone receptor membrane component 1 (PGRMC1).

Further, the present invention comprises the use of at least one reagent for influencing, in particular increasing or inhibiting, the abundance and/or activity of isoforms of proteins for diagnosis and/or therapy of diseases associated with aberrant biological phenotypes; wherein said proteins are involved, in particular differentially involved, in protein interaction or multi-protein complexes with either at least one isoform of phosphorylated or non-phosphorylated progesterone receptor membrane component 1 (PGRMC1)

According to a preferred embodiment of the invention said diseases, in particular subgroups thereof, comprise cancer, especially breast cancer or prostate cancer, neurodegenerative diseases, infertility, inflammatory, immunological, respiratory, pulmonary diseases, and/or diseases associated with the rate of biological aging or with beneficial or detrimental alterations of the level of the process of autophagy.

In the following the aspects of the present invention are explained in detail:
PGRMC1 interacts with both lnsig-1 and Scap1, which are involved in the cholesterol-dependent regulation of the cellular location of sterol regulatory element binding protein (SREBP). In the presence of cholesterol SREBP is retained in the endoplasmic reticulum, whereas it is escorted to the trans-Golgi network and proteolytically processed to release a cytoplasmic fragment which translocates to the nucleus and activates sterol regulatory element (SRE) driven genes. PGRMC1 probably senses levels of cholesterol or its metabolites in the endoplasmic reticulum [18], and therefore regulates this manifestly pleiotropic system.

The presence of conserved YXX(φ) consensus motifs (where φ represents an aliphatic amino acid) in PGRMC1 implicates the protein with an active role in membrane trafficking, since these motifs targets proteins for endocytosis [18;19]. PGRMC1 itself can translocate between endoplasmic reticulum, the plasma membrane, and the cytoplasm under various conditions, and because it acts as a steroid/cholesteroid sensor and contains regulatory protein interaction motifs that are regulated by phosphorylation [18], it is ideally situated to target interacting proteins to various cellular destinations. Therefore a brief consideration of membrane trafficking is appropriate.

Proteins that are newly synthesised within the endoplasmic reticulum may be either retained there, or they may be directed towards the trans-Golgi network. From there, one pathway leads to budding of vesicles that are eventually targeted to the plasma membrane. During the early 1990s it emerged that caveolae were characterized by the presence of scaffold proteins of the caveolin family and were involved in signal transduction and endocytosis/exocytosis processes. Caveolae are small cave-like invaginations of the plasma membrane induced by self-associating caveolin scaffold networks, most of which sequester cholesterol, glycosphingolipids and sphingomyelin as core lipid components to generate a membrane region where signalling complexes can preferentially accumulate and assemble at the surface of the cell. (Cells also contain low cholesterol caveolae. [20]) Membrane microdomains with similar biochemical properties and cell biological functions but lacking caveolin were later defined and became known as "rafts" or "lipid rafts", of which caveolae membranes form a subclass (for reviews see [20-22]). Proteins on the plasma membrane can be internalised by endocytosis via localisation to invaginating caveolae or larger and more electron dense clathrin-coated pits [23], which reflect the membrane composition of rafts.

Following internalisation by endocytosis, cell surface proteins are precisely sorted in endosomes of the endosomal compartment towards alternative subsequent fates. Some of them are recycled back to the cell surface whereas others are targeted to late endosomes which terminate in lysosomes for proteolytic degradation. Yet other proteins are directed towards multivesicular bodies, where luminal proteins are invaginated into the lumen of the mother vesicle and pinched off to form new vesicles within the lumen of the mother vesicle. The outer membrane of the resulting multivesicular body can fuse with the plasma membrane to release the internal vesicles which are then known as exosomes. This represents a novel pathway of secreting topologically cytoplasmic proteins to the extracellular space of living cells. Ubiquitination of target proteins provides a discriminatory sorting signal during this process. Polyubiquitin groups target cargo proteins to the lysosome, whereas one or more mono-ubiquitin groups direct proteins towards the multivesicular body fate. Ubiquitin-responsive sorting systems containing various proteins with the Ubiqitin Interacting Motif are located in sequential compartments along the secretory and endocytic pathways, and these probably exert their functions in a highly coordinated manner. Furthermore, appropriate ubiquitination of proteins in the late secretory pathway can also target proteins directly to the endosomal compartment without prior cycling to cell surface, or towards the multivesicular body pathway rather than the cell surface. The enrichment of cholesterol lipid rafts at the plasma membrane and in intracellular membrane regions, at least as early in the secretory pathway as the Golgi apparatus, facilitates this sorting process. In fact caveolin is also involved in the direct transport of cholesterol by cytoplasmic diffusion from the endoplasmic reticulum to the cell membrane. Thereby, ubiquitin and cholesterol are central in the control of endocytic sorting, and they are thought to act cooperatively (reviewed in [20;23]). That these processes can manifest severe pathologic aberrations in disease is obvious from the suitability of various E3 ubiquitin ligases, which add ubiquitin to specific target proteins, as cancer targets and biomarkers [24].

PGRMC1 can participate in this system in three ways. Firstly, by affecting cholesterol levels it can modulate the properties of lipid rafts, and thereby exert profound effects on cellular signalling. Secondly, by leaving the endoplasmic reticulum and entering the vesicular trafficking system it can direct interacting proteins to various different locations. Thirdly, its interaction partners can be regulated by differential phosphorylation of its protein interaction sites. Thus, PGRMC1 occupies a nexus function integrating cellular regulation whose influence ranges from the extracellular space, across the cell surface, through various regions of the cytoplasm and into the nucleus.

DCC was originally cloned as a potential tumour-suppressor protein from colorectal cancer, since it was lost by permissive cancers. Netrin receptors are now recognised as belonging to the class of 'dependence receptors' which create a cellular state of dependence on their respective ligands by inducing apoptosis in the absence of ligand, and the Netrin system has been recognised as being prominently important for tumour biology [34-36]. The cytoplasmic region of DCC does not contain any catalytic domain but possesses three conserved regions known as P1, P2, and P3. These interact with other receptors to form homo- or heterodimers important for netrin-1 function. The P3 domain is responsible for receptor dimerisation upon ligation which is required for neurite outgrowth. The P3 domain also interacts with the receptor Robo1 which is required for Slit-induced silencing [30;37-40]. Myosin X (Myo X) binds to the P3 domains of Neogenin and DCC, and is involved in the redistribution of DCC on the cell surface into a punctuate distribution. Myo X was required for neurite extension in response to Netrin where it enhanced the formation and extension of filopodia [41].

The absence of Netrin ligand induces self-activation of its receptor and the subsequent proteolytic processing of the receptor triggers apoptotic cell death. Ligand binding blocks the proteolytic processing and the proapoptotic activity of the receptor [31]. In the absence of Netrin, DCC indirectly recruits caspase 9 which activates caspase 3 or an unknown protease that in turn cleaves DCC [34;36]. At least two genetically dissectible signal pathways activated by DCC are conserved with Nematodes, involving Rac and the actin-binding abLIM on one hand and Enabled signalling on the other [42]. Netrin ligation to DCC activates ERK kinases which are recruited to the DCC receptor complex and are ultimately involved in axon migration since specific ERK-kinase inhibitors attenuate migration [43]. In the presence of Netrin, three pathways have been implicated in transducing cytoplasmic signals in mammalian cells, however under what circumstances any or all of these may act in native tissues remains unclear. Firstly, AKT2 may be activated by a protein called APPL (Adaptor protein containing pH domain, PTB domain and leucine zipper motif 1; Also called DIP13a; UniProt ID Q9UKG1) in conjunction with phosphatidylinositol 3-kinase (Pl3K). AKT signalling is known to be anti-apoptotic. Secondly, DCC directly binds and inhibits caspase 3, which accordingly cannot cleave DCC. A third mechanism may involve the activation of ERK kinases and concomitant survival signals discussed above (reviewed in reference [36]). Other molecules involved in DCC signalling include a DCC-interacting complex including FAK and FYN kinases through the P3 region, as well as another involving Cdc42, Rac1, Nck1, and Pak1 (reviewed in references [34;37]). Phosphatidylinositol transfer protein-alpha (PITPalpha) binds to both Neogenin and DCC upon ligation of Netrin. This stimulates the binding of PITPalpha to phosphatidylinositol-5-phosphate (PI5P), increasing its lipid-transfer activity and elevating the level of hydrolysis of phosphatidylinositol bisphosphate (PIP2). PITPalpha activity is required for the extension of elongating axons [44]. PIP2 hydrolysis in cortical neurons is mediated by DCC, but not Neogenin or the coexpressed Unc5 family receptor Unc5h2. Netrin-1 induces tyrosine phosphorylation of DCC near the P3 domain which was associated with phospholipase Cgamma tyrosine phosphorylation and activation. Furthermore inhibition of PLC activity attenuates Netrin-induced cortical neurite outgrowth [37].

Caspase activation in response to DCC by Netrin is essential for the attraction to Netrin during axonal guidance, and does not induce apoptosis. This occurs in the axon growth cone and involves the activation of p38 MAPK which locally activates the proteasomal protein degradation pathway that is essential for cytoplasmic reorganisation associated with attraction or repulsion of the growth cone. Caspase 3, but not Caspase 9, is involved in this process.

The response of neurons to Netrin induced by DCC is dependent upon not only the presence of members of the Unc5 and Robo receptor families, but also upon their prior state of activation, and may range from chemoattraction, chemorepulsion, migration, adhesion, or silencing, to apoptosis (reviewed in references [34;45]). For instance, PKC-mediated signalling after binding of Netrin to cells expressing DCC and Unc5A leads to endocytocytosis of Unc5A, changing the cell surface composition of receptors, and causing the axonal response to Netrin to change from growth cone collapse and chemorepulsion to attraction. This demonstrates that the response to Netrin can be dynamically altered over relatively short intervals by protein-directed alterations to cellular activity [46].

Metalloproteases potentiate Netrin-mediated axon outgrowth, suggesting that metalloproteases modulate responsiveness to Netrin by affecting the number of functional receptors on axonal membranes. Normal proteolytic attrition limits the number of functional DCC receptors available to establish active Netrin complexes on extending axons. Netrins intruigingly possess a region with striking similarity to tissue inhibitors of metalloproteases. Therefore metalloproteases are not simply involved in reorganising the extracellular matrix, but also in directly modulate the signals being transmitted through various Netrin receptors [47;48]. Subsequently to cleavage of the extracellular domain by an extracellular protease, the residual 'membrane stub' of the DCC receptor, which consists of cytoplasmic and transmembrane domains, can be cytoplasmically cleaved by the presenilin-gamma-secretase complex, which is also responsible for the processing of several type I membrane proteins including Amyloid Precursor Protein and Notch. Gamma-secretase cleavage of cytoplasmic DCC generates a derivative termed DCC-intracellular domain (ICD) which can translocate to the nucleus and that has an intrinsic transcriptional activation domain in the yeast two hybrid system [49], however no transcriptionally activated target genes have been reported. Presenilin cleavage of the intracellular domain teminates signalling from DCC, and this attenuates receptor-mediated intracellular signaling pathways that are critical in regulating glutamatergic synaptic transmission and memory processes [50]. Thus DCC/Netrin signalling governs not only one-time migrational behaviour in neurons, but is also dynamically involved in ongoing essential neuronal functions including those involved in memory formation. Indeed PGRMC1 has been implicated in the response of neurons to progesterone, including survival and dendritic growth, spinogenesis and synaptogenesis [51;52]. Therefore pharmaceutical reagents that affect PGRMC1 function can be employed in the treatment of memory disorders as well as cancers of the nervous system.

Gamma-secretase associates in a cholesterol-dependent manner into lipid rafts of post-Golgi and endosomal identity, whereas most of its substrates are spatially segregated into cholesterol poor and detergent soluble non-lipid raft membranes. Yet gamma-secretase processing of substrates such as DCC and N-cadherin c-terminal fragments that have already been extracellularly proteolysed occurs in non-raft fractions in embryonic brain. Furthermore, signalling caused by regulated intramembrane proteolysis of specific substrates is regulated by the partitioning of active gamma-secretase into cholesterol rich lipid raft domains, and can be perturbed by disruptions to the cholesterol balance [53]. The intimate association of PGRMC1 with cholesterol metabolism and its feedback control has been reviewed [18].

An intracellular pool of DCC exists in vesicles that are targeted to the plasma membrane upon PKA activation. Translocation of DCC depends upon active adenylate cyclase, PKA, and exocytosis. Therefore netrin-1 can increase the level of cell surface DCC via cAMP and PKA, potentiating the response to netrin-1 [54]. PGRMC1 interacts with DCC and has been implicated to be involved in vesicle trafficking [18;19], thereby post-translationally modulating the availability of its interaction partner DCC (and Neogenin) at the cell surface.

In this context it is pertinent to note that DCC cleavage by caspase 9 to induce apoptosis in the absence of its Netrin ligand requires the localisation of DCC to cholesterol rafts [55]. Thereby differences in PGRMC1 phosphorylation can not only direct the localisation of DCC from intracellular vesicles to the cell surface, but also potentially affect the localisation of DCC in cholesterol-rich lipid-rafts that are required to induce apoptosis.

### The Netrin signalling system in non-neurons

The biological processes affected by the Netrin/Neogenin system include a broad range of epithelial morphogenesis and maintenance processes in addition to neuronal guidance and survival [34;56;57]. Netrin is not only a neural attractant but also an angiogenic factor. Neogenin mediates Netrin signaling in vascular smooth muscle cells, whereas another unidentified receptor mediates the proangiogenic effects of Netrin-1 on vascular endothelial cells [34;58]. DCC-dependent vasculogenesis after Netrin exposure is induced via ERK kinases upstream of Endothelial Nitric Oxide Synthase (eNOS), an enzyme from mammalian blood vessels which leads to NO' production in aortic endothelial cells. This in turn induces migration and cell division leading to vessel initiation and extension [59]. The modulation of DCC activity by PGRMC1 is especially interesting in this context because of the involvement of PGRMC1 in the mevalonate pathway leading to cholesterol synthesis and production of prenyl lipids (reviewed by reference [18]). PGRMC1 is explicitly required for cholesterol synthesis [60]. Statins inhibit the enzyme 3-hydroxy-3-methylglutarate-CoA reductase (HMG-CoAR) which is an SREBP-regulated gene that is the rate limiting enzyme in the mevalonate pathway. Statins can enhance NO'-dependent angiogenesis by reducing caveolin-1 abundance and its inhibitory effect on eNOS, and this mechanism operates in tumours [22;61]. Thereby PGRMC1 is able to influence the angiogenic effect of DCC-dependent NO* production by modulating both DCC function and the mevalonate pathway which upregulates caveolin and thereby down-regulates eNOS activity. Since eNOS activity is a critical factor associated with inflammatory diseases [22], reagents directed against PGRMC1 are therefore also suitable for the treatment of inflammatory diseases such as atherosclerosis, as well as cardiovascular diseases.

Skeletal muscle cells undergo terminal differentiation whereby they undergo cell fusion to form polynuclear syncitial myotubes. The Neogenin/Netrin system is involved in the generation of signals that lead to myotube formation, where active Neogenin and Netrin are present in a trans-cellular protein complex containing cadherins [62;63]. Cadherins are involved in cell-cell contacts such as through adherens junctions, which mediate contact growth inhibition by limiting cytoplasmic catenin levels while simultaneously generating survival signals. They are also crucial for correct organization of nascent vessels in angiogenesis (reviewed in reference [64]). Based upon phylogenetically conserved localisation of Netrin to the cell surface of cells that express it, Kennedy proposed a role of Netrin/DCC in the maintenance of cell contacts and intercellular interactions in multiple tissue types as early as 2000 [30]. This is strengthened by the observation that DCC is involved in the regulation of cell adhesion in human colorectal cancer derived HT-29 cells, where it associates with ezrin, a linker of membrane to the cytoskeleton [65]. Loss of this regulation is affected when DCC is deleted in colorectal cancer. Thus the survival signals supplied by these dependence receptors are related to contact growth inhibition and linked cytoskeletal organisation, which represents active supervision of static tissue conditions.

Hemojuvelin (HJV) is a member of the RGM family that interacts with Neogenin. These two proteins are involved in a system involving ferritin that leads to increase in cytoplasmic iron levels of Neogenin-expressing cells [57,66]. This is important since iron levels increase the rate of production of reactive oxygen intermediates, which not only influence cellular redox status but also act as transducers in associated signal transduction [67]. The activation of eNOS in vascularisation has been described above.

The Netrin signalling system in breast morphogenesis

The Netrin/Neogenin system directs the developing mammary terminal end buds, which are highly proliferative structures found at the invading edge of developing mammary glands [68]. The mammary end bud is a dynamic system, whose motility requires the successful integration of systemic and local mammotrophic influences coordinating ductal growth regulation, extracellular matrix remodeling, and cell adhesion in the inner end bud. Stem cells present in the ductule walls can also be induced to initiate new end buds to form branches, both during pubertal development and during pregnancy. This system is under hormonal control, responding to progesterone among other hormones. Netrin is expressed on the extracellular surface or is present in the adjacent extracellular matrix of luminal epithelial cells of the end buds. Neogenin is expressed in the immediately overlying cap cells. Null mutations in either mouse gene cause slower end growth and more disorganised end buds: particularly caused by loss of adhesion between luminal epithelial and cap cells, and apoptosis of the disoriented cap cells [69].

The role of the Netrin/Neogenin system in controlling the invasive behaviours of mammary epithelial cells was further characterised by Strizzi et al. [70] who examined Cripto-1 signalling. Cripto-1 is a member of the EGF family that signals through Nodal, a member of the TGFβ receptor superfamily, to induce Netrin slightly and Neogenin markedly. This is associated with increased proliferation, migration, invasion and colony formation by epithelial cells in 3D matrices, which is accompanied by increased AKT phosphorylation with presumed involvement of PI3K. By contrast, treatment with Netrin alone leads to presumed signalling through the Unc5H Netrin receptor and a concomitant anti-invasive phenotype. A neutralising antibody against Unc5H increased the degree of invasiveness in Netrin-treated cells whereby a neutralising antibody against Neogenin decreased the number of invading cells in an in vitro assay [70]. Therefore increased abundance of Neogenin relative to Unc5H was involved with orchestration of the invasive response to Netrin. The ability of PGRMC1 to modulate the availability or function of Neogenin at the cell surface thereby contributes to the hormone responsiveness of this system.

The effect of constitutive Cripto-1 activation described above resembles the phenomenon of epithelial-mesenchymal transition (EMT) [70]. This is a genetic program of mammalian cells characterized by loss of cell adhesion, repression of E-cadherin expression, and increased cell mobility. EMT is essential for numerous developmental processes including mesoderm formation and neural tube formation. Initiation of cancer metastasis involves tissue invasion, which has many phenotypic similarities to EMT, including a loss of cell-cell adhesion mediated by E-cadherin repression and an increase in cell mobility. EMT has been explicitly linked to the ability of breast cancer cells to enter the circulation and induce metastases [71-74]. The above identifies Neogenin as an orchestrator of EMT in breast cancer. Furthermore, ER- tumours exhibit a basal rather than luminal phenotype accompanied by a higher propensity to seed metastases and poorer patient prognosis [11;75].

Hypophosphorylated isoforms of PGRMC1 were more abundant in ER-tumours (PCT/EP2006/009351) and the phosphorylation sites are present in protein regions that are involved in protein interactions [18]. Therefore PGRMC1 interacts with a different set of cellular proteins in ER- tumours, which explains the different subcellular localisation of PGRMC1 between tumours possessing or lacking the ER (PCT/EP2006/009351). Neogenin interaction with PGRMC1 is obvious from conservation of this biological system with nematode homologues [19]. The ability of PGRMC1 to modulate the availability or function of Neogenin at the cell surface thereby identifies PGRMC1 as a key target for cancer therapy. This carries the further advantage that EMT is a non-essential function for overall survival in adults, and therefore this system is amenable to pharmacological intervention with only moderate side-effects.

This identifies a biological system whereby breast cells can be induced to exit the dormant Go cell cycle and begin to both invade adjacent tissue and enter the cell division cycle. Both of these processes are deregulated in pathological cancer cells, emphasising that deregulation of the Netrin signalling system is of paramount importance to cancer. Indeed normal breast tissues express higher levels of Neogenin than infiltrating ductal carcinoma [76], strongly implicating perturbations in the Neogenin/Netrin system in tumour neogenesis. Furthermore involvement of PGRMC1 in this mechanism of action is fully concordant with the observed late induction of PGRMC1 in a model of spinal chord injury and wound response, at a time when tissue regeneration was underway [77].

The above demonstrate a plurality of examples of modulation of the Netrin signalling system to control cellular behaviour, and it is remarkable to find avenues for manipulation by PGRMC1 at several levels. In this context it is notable that the PGRMC1-interacting protein PAIRBP1 binds to the cyclic nucleotide-responsive sequence in the Type-1 plasminogen activator inhibitor mRNA [78]. This serine protease inhibitor (serpin) can affect the activity of extracellular proteases that are important for not only extra cellular matrix dissolution and tissue remodelling but also for receptor proteolysis. Furthermore, Netrin/DCC is known to be intimately associated with cAMP signalling [43;79-83], and PAIRBP1 binds to cyclic nucleotide-responsive regulatory sequences in mRNA. PGRMC1 can sequester PAIRBP1, thereby modulating this regulation. Therefore PGRMC1 manipulates the Netrin signalling pathway at various stages, disclosing multiple and previously unrecognised levels at which PGRMC1 and its differentially phosphorylated isoforms can influence the motility and survival of tumour cells . These are biological processes that are extremely relevant to tumour progression and metastasis.

For a more detailed description of the present invention, reference will now be made to the accompanying table and figures:

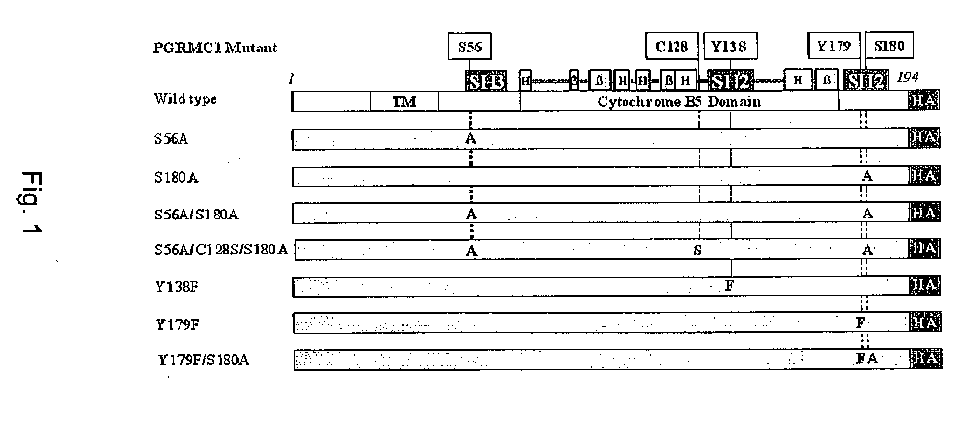

- **Fig. 1**: This figure shows the PGRMC1 proteins expressed from stably transfected plasmids as described in PCT/EP2006/009351. Amino acid numbering is according to the PGRMC1 amino acid sequence of human Uniprot entry 000264, which does not include the initiator methionine. Uniprot Q6IB11 corresponds to the same sequence with initiator methionine included, where mutated human PGRMC1 amino acids would be numbered as Ser57, Cys129, Tyr139, Tyr180, Ser181, etc.

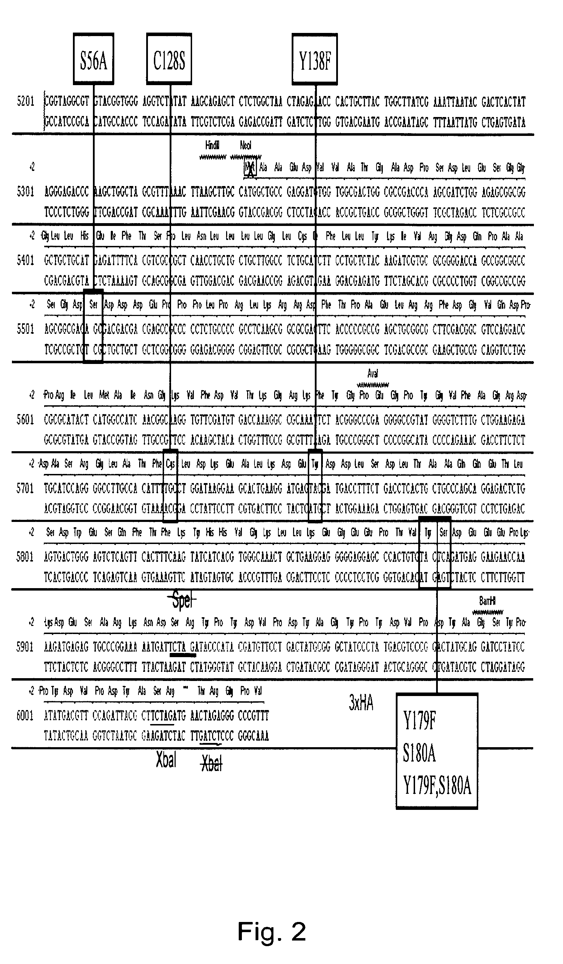

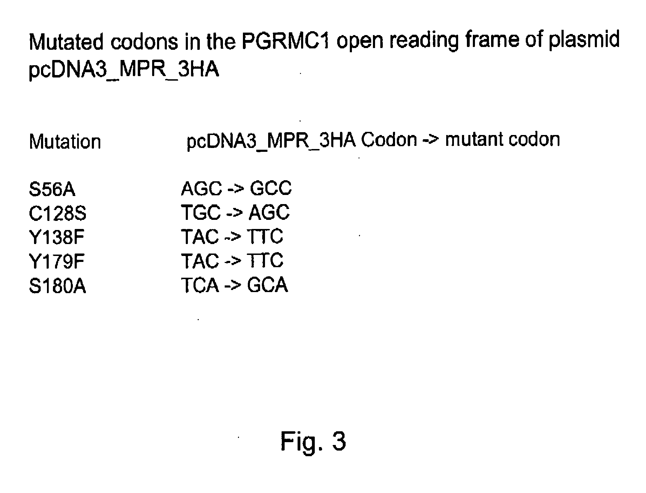

- **Fig. 2**: The sequence of wild type PGRMC1 (also known as mPR) is indicated, as well as location of site directed mutations of Figure 3.
- **Fig. 3**: Site directed mutations introduced into the open reading frame of plasmid pcDNA3_MPR_HA to generate the amino acid mutations indicated. The positions of each codon within the PGRMC1 open reading frame are shown in Figure 2.

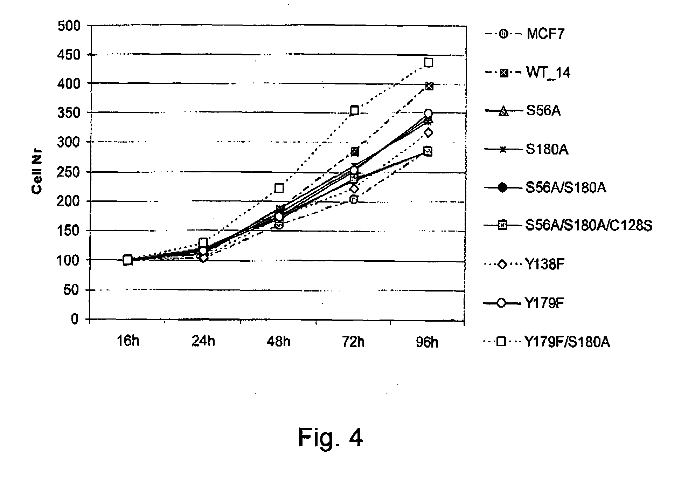

- **Fig. 4**: Growth kinetics of PGRMC1 mutants. 5000 cells were plated into 96 well plate in triplicates and incubated at 37°C/5% CO2 in RPMI with 10% FCS. At indicated time points supernatant was decanted and ATP-Assay was performed. This figure shows that the Y179F/S180A mutant proliferates faster than other cells in the assay. The error bars (standard error) are shown, yet are in all cases smaller than the size of the symbols of plotted points.

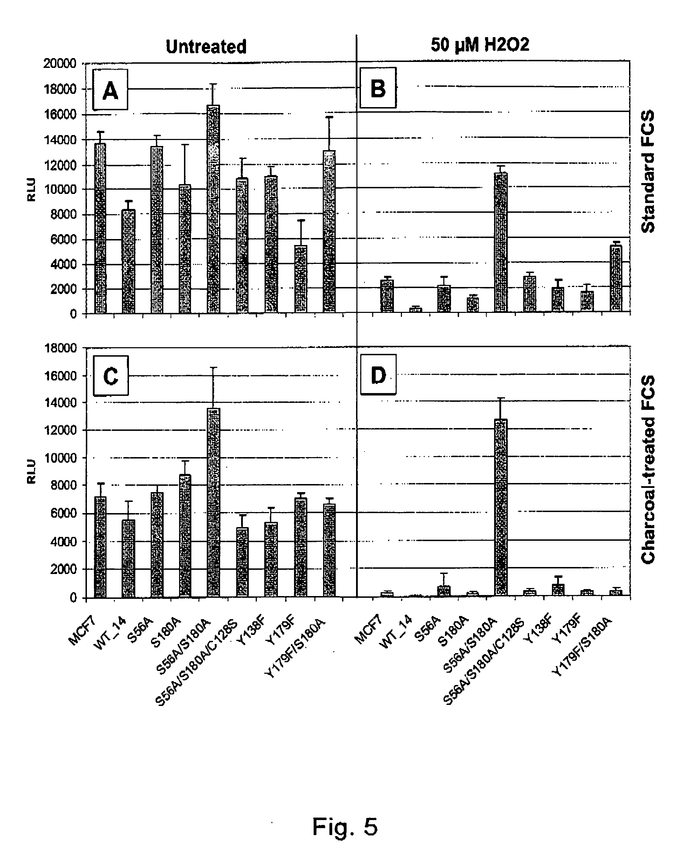

- **Fig. 5**: Effects of PGRMC1 mutants on cell viability after H2O2 treatment. A) Cell lines were grown in RPMI with 10% FCS. B) Cells grown in RPMI with 10% FCS with addition of 50µM H2O2. C) The analogous experiment to A, performed using charcoal-treated FCS to remove steroids and cholesterol. D) The analogous experiment to C, with addition of 50µM H2O2. This figure shows that S56A/S180A and Y179F/S180A are resistant to H2O2-induced cell death in medium containing normal serum, whereas only S56A/S180A is resistant in medium containing charcoal-treated serum, which removes hydrophobic molecules such as steroids. RLU = Relative light units, whereby ATP is detected by a fluorescence reaction.

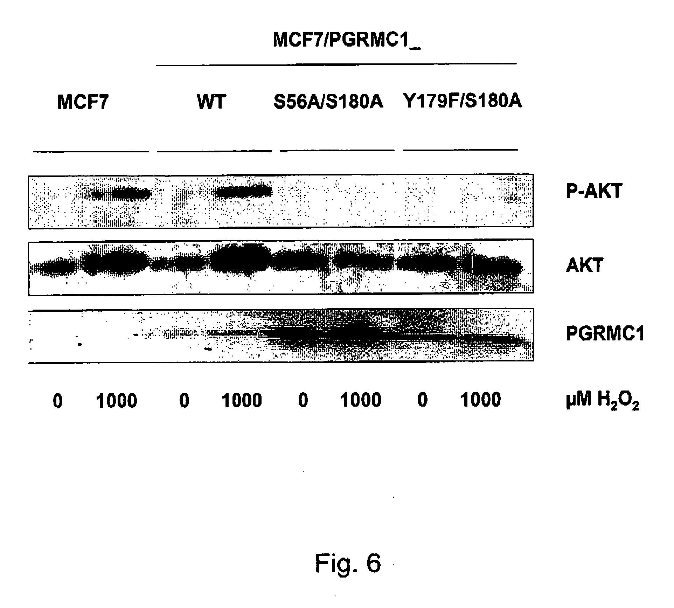

- **Fig. 6**: PGRMC1 influences phosphorylation of AKT after H2O2 treatment. The figure shows a western blot of equal total protein amounts (10µg/lane) from cel extracts fo the indicated cells. Antibodies employed were specific for phosphorylated AKT (top), for the polypeptide backbone of AKT (middle), or for the HA-tag on the respective exogenously expressed PGRMC1 constructs.

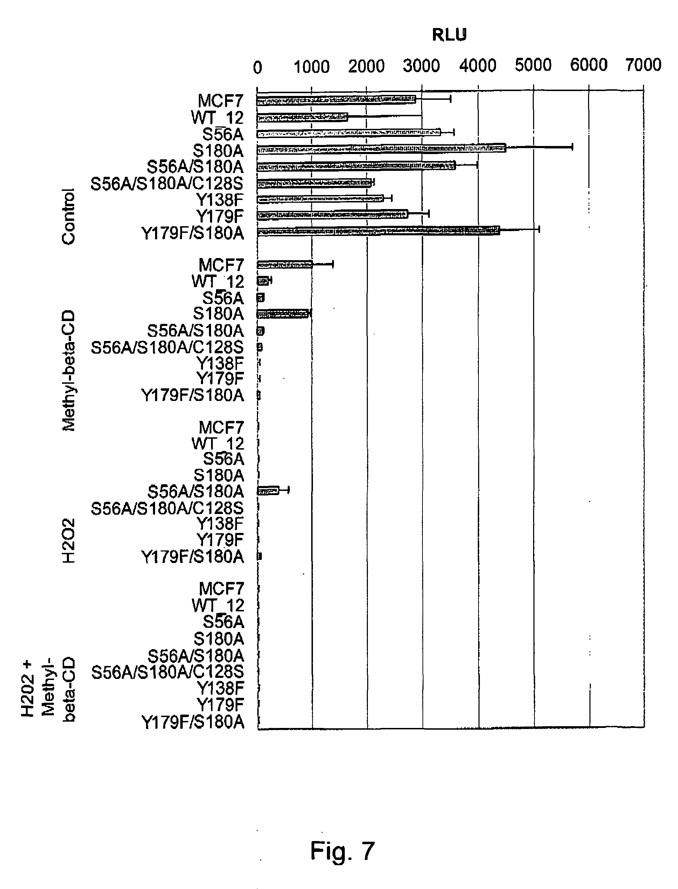

- **Fig. 7**: PGRMC1 S180A abrogates Methyl-β-cyclodextrin-induced death.
This figure shows that the response to Methyt-β-cyciodextrin (Methly-beta-CD) is different than to H2O2. All PGRMC1 mutants except S180A induce enhanced cell death. Methly-beta-CD was purchased from Sigma (#C4555) and administered as described [53]. 5000 cells were plated into 96 well plate in triplicates and incubated at 37°C/5% CO2. On day two medium was changed to RPMI/Hyclone charcoal-treated FCS. On day 3 cells were washed with PBS and incubated with 5mM Methyl-beta-Cyclodextrin in RPMI/Hyclone charcoal-treated FCS for 2h. Then the ATP-Assay was performed.

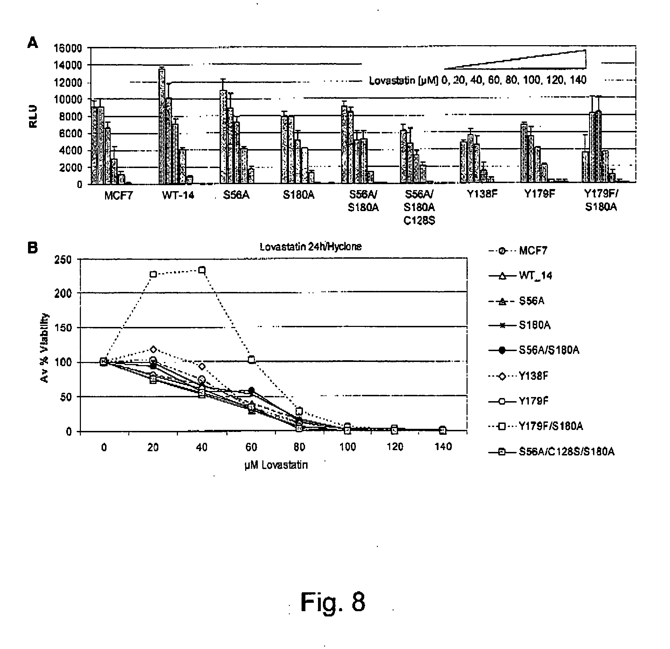

- **Fig. 8**: PGRMC1 modulates the mevalonate pathway. A. Replicate values +/- standard error of cell survival assays under the indicated concentrations of lovastatin for the PGRMC1 mutants. Cells are grown in RPMI medium with charcoal-treated FCS which is depleted in cholesterol. RLU = relative light units. B) Average viability of mutants from A, normalised to growth conditions in the absence of lovastatin (100% at zero concentration). Lovastatin was purchased from Sigma (#M2147) and administered as described [87]. 5000 cells were plated into 96 well plate in duplicates and incubated at 37°C/5% CO2 On day 2 cells were incubated with increasing levels of lovastatin for 2 days, followed by cell harvest and performance of the ATP-Assay.

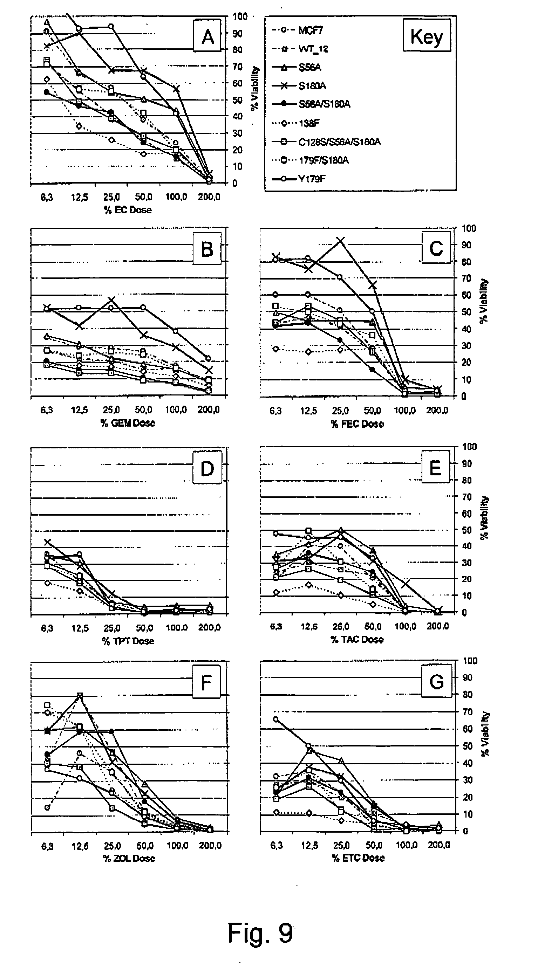

- **Fig.9**: Response of cells expressing PGRMC1 mutants to cytostatic reagents. The clinical cytostatics used were: A) EC: Epirubicin/Maphosphamid; B) GEM: Gemcitabine; C) FEC: 5-Fluoruracil/Epirubicin/Maphosphamide; D) TPT: Topotecan; E) TAC: Taxol/Adriamycin/Maphosphamide; F) ZOL: Zoledronic acid (Zometa); G) ETC: Epirubicin/Taxol/Maphosphamid. Concentrations are given as percent respective clinical Test Drug Concentration (TDC) dosage. The key shows the identity of mutants from Figure 1-Figure 3. 5000 cells were plated into 96 well plate in duplicates and incubated at 37°C/5% CO2 for 7 days in RPMI with 10% FCS. After 7 days the cells were harvested and ATP-Assays for viability were perfomed (on day 10 of the experiment). Each point is the average of replicate determinations. The 100% TDCs for the cytostatics were 5-Fluoruracil: 170 µM; Epirubicin: 0.9 µM; Gemcitabine (Gemzar): 219 µM; Taxol: 15.9 µM; Topotecan: 1.5 µM; Cyclophosphamid/Maphosphamid: 11.5 µM; Zoledronic acid: 34.5 µM; Doxorubicin/Addamycin: 0.9 µM.

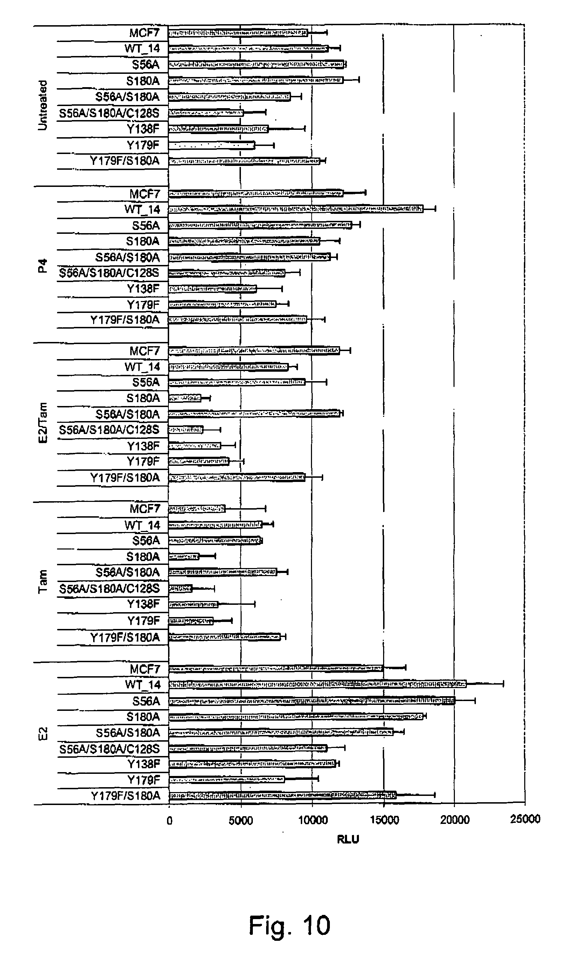

- **Fig. 10**: Effects of hormone treatment on PGRMC1 mutants. 5000 cells were plated into 96 well plate in triplicates (Tamoxifen only in duplicates!) and incubated at 37°C/5% CO2. On day two medium was changed to RPMI with 10% charcoal-treated Hyclone FCS and hormones/anti-hormone were added on day three. The indicated treatments were P4: progesterone (10⁻⁷ M); E2: estrogen (10⁻⁹ M); Tam: tamoxifen (10⁻⁸ M). After growth in the presence of hormones for 5 days an ATP-Assay was performed.

In PCT/EP2006/009351 an expression plasmid encoding the human PGRMC1 open reading frame with a C-terminal influenza virus hemaglutanin (HA) epitope tag was used as a template to construct specific directed amino acid mutations in PGRMC1 (Figure 1 - Figure 3). The construction of PGRMC1 plasmids and the establishment of individual sub-clones expressing each PGRMC1 variant have been described (PCT/EP2006/009351). The methods, plasmids, mutations in PGRMC1 and PGMRC1 variants disclosed in PCT/EP2006/009351 are hereby incorporated by reference. Same applies to all other cited references which are hereby incorporated into this document, too. Stable transfectants of the breast cancer cell line MCF7 were obtained expressing all mutants. MCF-7 cells were maintained in RPMI medium containing 10% fetal calf serum (FCS), 2 mM L-glutamine, and penicillin and streptomycin. PGRMC1 expression in all cell lines was confirmed by Western blot using an HA-specific monoclonal antibody [84]. Here the effects of those mutations on the response of cells to various-stimuli are analysed.

We estimated cell viability using an assay which utilized the fluorometric determination of ATP levels as an indicator of cell viability. The assay for cell viability was performed using the ATP-TCA kit (TCA-100; DCS Innovative Diagnostik Systeme, Hamburg, Germany) as follows: cells were incubated with 50µl tumor cell extraction reagent and 50µl RPMI-medium for 30 min at RT. Then 50µl supernatant was transferred into a 96 well plate and 50µl chemoluminescence reagent was added. Chemoluminescence was quantitated using a luminescence reader (Berthold, Germany). The fluorometric readout is presented in relative light units (RLU) in the figures, whereby higher RLU values are associated with higher ATP levels and thereby higher levels of cell viability.

First we measured whether any of the mutations in PGRMC1 affected the kinetics of growth in culture of the cell lines. The Y179F/8180A mutant proliferated more quickly than other cell lines as also did WT PGRMC1 to a lesser degree Figure 4. This indicates that tyrosine phosphorylation of Y179 can exert an inhibitory effect on proliferation, as can serine phosphorylation of S180.

Expression of PGRMC1 in MCF-7 cells sensitized the cells to death following long-term/low dose or short-term/high dose treatment with hydrogen peroxide. Cell death did not occur through a typical apoptotic mechanism and corresponded with hyperphosphorylation of the AKT kinase protein [84]. Cells expressing all PGRMC1 proteins in our panel of mutants exhibited comparable growth in cell culture medium containing normal fetal calf serum (FCS) as the parental MCF7 cells (Figure 5A). Treatment of MCF7 cells with 50µM H2O2 reduced cell viability, and exogenous expression of the wild type PGRMC1 protein (designated WT_14 in the figures) further exacerbated this reduced viability (Figure 5B; compare MCF7 with WT_14) which was consistent with the expected situation as reported [84]. Notably, the viability of the S56A/S180A mutant, and to a lesser extent of the Y179F/S180A mutant, were considerably greater than the viability of MCF7 cells, or cells expressing the WT PGRMC1 protein (Figure 5B). This trend was also observed at 100µM H2O2 (data not shown). This result is notable because both of these PGRMC1 mutants produced unusually low numbers of surviving colonies in one round of stable selection (PCT/EP2006/009351).

An experiment was performed with replicate design, but using charcoal/Dextran-treated 10% FCS (Hyclone SH30068). Charcoal-stripping removes steroid hormones and other components from the FCS. All cell lines were able to grow in this medium (Figure 5C), however the degree of viability was less in the presence of H2O2 for all cells with the exception of the S56NS180A double mutant (Figure 5D). Therefore charcoal-treatment of FCS removes some component which is necessary for the survival of PGRMC1 mutant Y179F/S180A, but not for S56A/S180A. Cysteine 128 was essential for this vital function of the S56A/S180 mutant, and it is most probable that dimersiation via a cystine-mediated disulfide bond [18] is required for the rescuing function. Taken together, the growth advantage of the Y179F/S180A mutant translates into a more robust response to H2O2 stress, however this requires some hydrophobic component (such as cholesterol) removed by treatment of FCS with charcoal. The S56A/S180A is able to circumvent H2O2-mediated cell death even when growing in charcoal-treated FCS.

H2O2-induced cell death of MCF7 cells and MCF7 cells expressing exogenous PGRMC1 was associated with phosphorylation of AKT, as reported in the literature [84], therefore we examined AKT phosphorylation of these mutants in Figure 6. Strikingly, neither of the mutants that survived H2O2 treatment in Figure 5B exhibited phosphorylation of AKT upon H2O2 treatment, even at 1 mM levels (Figure 6). Therefore these PGRMC1 mutants are able to exert profound effects on the intracellular signal transduction pathways activated in response to external stimuli, strongly supporting a role for differentially phosphorylated PGRMC1 in the biology of breast tumours.

Because PGRMC1 has been implicated with a regulatory role in cholesterol metabolism, we assayed the effects of treating the cell lines with methyl-β-cyclodextrin (Methly-beta-CD), a drug known to selectively deplete biological membranes of cholesterol (Figure 7). Whereas the S56A/S180A mutant was once more resistant to H2O2, the expression of all PGRMC1 mutants except S180A drastically reduced cell viability relatively to MCF7 control cells. This demonstrates that PGRMC1 induces a biological response in the absence of cholesterol that compromises cell viability. Serine 180 of PGRMC1 is required to induce the biological effect which kills cells in the absence of cholesterol. Therefore its phosphorylation is probably responsible for the relocalisation of PGRMC1 to a cellular compartment where the biological effect is propagated, possibly from the endoplasmic reticulum to the Golgi-apparatus-Depletion of cholesterol combined with H2O2 treatment was extremely toxic to all cells (Figure 7). These data are compatible with PGRMC1 monitoring cholesterol levels and inducing a biological response. Furthermore, phosphorylation of Ser180 is involved in the response to depleted cholesterol levels.

At low levels of cholesterol the SREBP protein is escorted from the endoplasmic reticulum to the Golgi-apparatus, where it is proteolytically cleaved to generate the SREBP transcription factor. This induces sterol response element (SRE)-driven genes including HMG-CoA-Reductase which activates the mevalonate pathway and enhances cholesterol synthesis (see [18] for references). Therefore we assayed the effect of lovastatin on the different mutants. Statins are inhibitors of HMG-CoA-Reductase, a rate limiting enzyme in the mevalonate pathway [85]. While the viability of all cell lines was reduced by high levels of lovastatin, the PGRMC1 Y179F/S180A exhibited improved viability at lower concentrations of lovastatin. The Y138F mutant also exhibited a slight trend in this direction, although it was not significantly different than the other clones (Figure 8). Interestingly the Y179F/S180A mutant exhibited somewhat lower ATP levels in the absence of lovastatin (Figure 8A), perhaps reflecting constitutive activation of the mevalonate pathway which has some negative effect on viability. Low concentrations of Lovastatin alleviated this effect, elevating ATP production and producing a relative increase in cell survival, whereas higher concentrations of Lovastatin were fatal. Y138F exhibits a slight trend in the same direction. Both of these mutations remove the phosphate acceptor of an SH2-domain target sequence, indicating that tyrosine phosphorylation of PGRMC1 Y179 (and perhaps also Y138) is necessary for interaction with other proteins that mediates repression of the mevalonate pathway.

Next we investigated the potential of PGRMC1 mutants to modulate the survival of cells in response to cytostatic reagents commonly used in chemotherapy (Figure 9). Strikingly, the mutants Y179F and S180A were often among the most viable cells in these assays, especially for treatment with EpirubicinlMaphosphamide (EC), Gemzitabine (GEM), and 5-Fluoruracil/Epirubicin/Maphosphamide (FEC). For the former two more than 30% of cells were viable even at 100% of the clinically employed concentration (Figure 9 A-C). These treatments consisted of Gemcitabine (a nucleotide analogue that interferes with DNA replication) and Epirubicin (a DNA intercalator that interferes with DNA replication) and Maphosphamide (an alkylating reagent). The FEC treatment also include 5-fluorouracil, another nucleotide analogue. These data provide insight into the mechanism of cell death evasion by PGRMC1, as well as indicating that certain classes of chemotherapeutic reagents will be more effective than others for different tumours containing differentially phosphorylated PGRMC1 protein. This emphasises the diagnostic utility of PGRMC1 phosphorylation.

Crudden et al. have demonstrated that depletion of PGRMC1 from cells by RNAi increases the susceptibility to chemotherapeutic reagents. Furthermore exogenous overexpression of PGRMC1 increased sensitivity to the DNA intercalating agent doxorubicin and the topoisomerase-I modulator camptothecin [86], which induces single strand DNA nicks transcribed genes. It has been suggested that modulation of cytochrome P450 enzyme activity may be responsible for altering the cytotoxicity of cytostatics. Our results demonstrate that the phosphorylation of PGRMC1 is crucial for the response to cytostatic agents employed in chemotherapy, yet the response is mediated by different mutants than the response to either cholesterol depletion or H2O2, clearly identifying PGRMC1 as a multi-functional protein which affects many different cellular responses. There is clearly more to the PGRMC1 response than cytochrome P450 regulation alone. Furthermore, the dynamic reversal of the state of phosphorylation provides the cell with an avenue to regulate these responses via PGRMC1, identifying this protein as a key therapeutic target molecule. Indeed, ER- tumours differ in the phosphorylation status of PGRMC1, and exhibit worse response to treatments including chemotherapy as discussed above.

Finally, we assayed the hormone dependence of various mutants, for which the results are shown in Figure 10. MCF7 cells express the classical (cytoplasmic) estrogen and progesterone receptors. Progesterone (P4) exerted a marginal proliferative effect on the PGRMC1-WT cell line relative to other cells, yet generally the viability of cells growing in the presence of charcoal-treated FCS was not affected greatly by progesterone. As expected, the viability of cells was improved when estrogen (E2) was added to the RPMI medium containing only charcoal-treated FCS, whereas addition of tamoxifen reduced viability. Tamoxifen (Tam) competes with estrogen for the ER to inhibit the transcription of estrogen-responsive genes, and estrogen could not rescue the impaired viability of cells in the presence of tamoxifen, as expected.

Surprisingly, whereas the expression of exogenous wild-type PGRMC1 did not markedly affect the response of MCF7 cells to Tamoxifen, several of the PGRMC1 mutants greatly reduced cell viability in response to tamoxifen with or without the presence of estrogen. Clone S180A proved to be highly susceptible to Tamoxifen, whereas clone S56A/S180A exhibited resistance to that reagent which was comparable to the wild-type PGRMC1 control or to native MCF7 cells. The reaction profile across the different mutants in Figure 10 demonstrates that the phosphorylation of PGRMC1 is required by MCF7 cells to survive in the presence of tamoxifen. Furthermore the requirement for multiple phosphorylation sites indicates that the coordinated phosphorylation and dephosphorylation of PGRMC1 is required for this protective phenotype. Cysteine 128, implying a redox-mediated step, was also essential for the ability to remain viable in the presence of tamoxifen as apparent from comparison of the S56A/S180A and S56A/C128S/S180A mutants in Figure 10.

These results unambiguously identify PGRMC1 as a cancer target capable of modulating cell survival following therapeutic treatment. Furthermore, it is obvious that dynamic flux in the state of phosphorylation of different sites of PGRMC1 is required for this vital or death-evading function. Therefore reagents capable of modulating the state of PGRMC1 phosphorylation will have a profound effect on cancer treatment, as well as the other indication areas of neural disorders, cardiovascular disease and inflammatory diseases where a role of PGRMC1 is obvious from the discussion above.

### Conclusions:

PGRMC1 phosphorylation mutants affect growth and survival properties of cells, and are responsive to modulators of cholesterol abundance. It must be remembered that the effects induced by these mutants can be modulated inside in cells in a regulated manner by selective phosphorylation and sub-localisation of PGRMC1 in response to various stimuli. This study identifies PGRMC1 as a central regulator of cellular response to external challenges. Although we demonstrate these results using MCF7 breast cancer cells, the mechanisms of PGRMC1 protein interaction and phosphorylation are general as evidenced by the descriptions above, and PGRMC1 is therefore suitable for the diagnostic and therapeutic treatment of not only cancer but also nervous system disorders, diseases of inflammation, and cardiocascular disorders.

### References.

[1] Jemal A, Siegel R, Ward E, Murray T, Xu J, Thun MJ: Cancer statistics, 2007. CA Cancer J Clin 2007;57:43-66.
[2] Fisher B, Jeong JH, Dignam J, Anderson S, Mamounas E, Wickerham DL, Wolmark N: Findings from recent National Surgical Adjuvant Breast and Bowel Project adjuvant studies in stage I breast cancer. J Natl Cancer Inst Monogr 2001;62-66.
[3] Osborne CK: Steroid hormone receptors in breast cancer management. Breast Cancer Res Treat 1998;51:227-238.
[4] Henderson IC: Risk factors for breast cancer development. Cancer 15-3-1993;71:2127-2140.
[5] Howell A, Osborne CK, Morris C, Wakeling AE: ICI 182,780 (Faslodex): development of a novel, "pure" antiestrogen. Cancer 15-8-2000;89:817-825.
[6] McKeage K, Curran MP, Plosker GL: Fulvestrant: a review of its use in hormone receptor-positive metastatic breast cancer in postmenopausal women with disease progression following antiestrogen therapy. Drugs 2004;64:633-648.
[7] Platet N, Prevostel C, Derocq D, Joubert D, Rochefort H, Garcia M: Breast cancer cell invasiveness: correlation with protein kinase C activity and differential regulation by phorbol ester in estrogen receptor-positive and -negative cells. Int J Cancer 2-3-1998;75:750-756.
[8] Thompson EW, Paik S, Brunner N, Sommers CL, Zugmaier G, Clarke R, Shima TB, Torri J, Donahue S, Lippman ME, Association of increased basement membrane invasiveness with absence of estrogen receptor and expression of vimentin in human breast cancer cell lines. J Cell Physiol 1992;150:534-544.
[9] Fuqua SA, Cui Y: Estrogen and progesterone receptor isoforms: clinical significance in breast cancer. Breast Cancer Res Treat 2004;87 Suppl 1:S3-10.
[10] Price JE, Polyzos A, Zhang RD, Daniels LM: Tumorigenicity and metastasis of human breast carcinoma cell lines in nude mice. Cancer Res 1-2-1990;50:717-721.
[11] Sorlie T, Perou CM, Tibshirani R, Aas T, Geisler S, Johnsen H, Hastie T, Eisen MB, van de RM, Jeffrey SS, Thorsen T, Quist H, Matese JC, Brown PO, Botstein D, Eystein LP, Borresen-Dale AL: Gene expression patterns of breast carcinomas distinguish tumor subclasses with clinical implications. Proc Natl Acad Sci U S A 11-9-2001;98:10869-10874.
[12] Sorlie T, Tibshirani R, Parker J, Hastie T, Marron JS, Nobel A, Deng S, Johnsen H, Pesich R, Geisler S, Demeter J, Perou CM, Lonning PE, Brown PO, Borresen-Dale AL, Botstein D: Repeated observation of breast tumor subtypes in independent gene expression data sets. Proc Natl Acad Sci U S A 8-7-2003;100:8418-8423.
[13] Abd El-Relidm DM, Pinder SE, Paish CE, Bell J, Blamey RW, Robertson JF, Nicholson RI, Ellis IO: Expression of luminal and basal cytokeratins in human breast carcinoma. J Pathol 2004;203:661-671.
[14] Nielsen TO, Hsu FD, Jensen K, Cheang M, Karaca G, Hu Z, Hemandex-Boussard T, Livasy C, Cowan D, Dressler L, Akslen LA, Ragaz J, Gown AM, Gilks CB, van de RM, Perou CM: Immunohistochemical and clinical characterization of the basal-like subtype of invasive breast carcinoma, Clin Cancer Res 15-8-2004;10:5367-5374.
[15] Weigelt B, Glas AM, Wessels LF, Witteveen AT, Peterse JL, van't Veer LJ: Gene expression profiles of primary breast tumors maintained in distant metastases. Proc Natl Acad Sci U S A 23-12-2003;100:15901-15905.
[16] Dvorak HF: Tumors: wounds that do not heal. Similarities between tumor stroma generation and wound healing. N Engl J Med 25-12-1986;315:1650-1659.
[17] Chang HY, Sneddon JB, Alizadeh AA, Sood R, West RB, Montgomery K, Chi JT, van de RM, Botstein D, Brown PO: Gene expression signature of fibroblast serum response predicts human cancer progression: similarities between tumors and wounds. PLoS Biol 2004;2:E7.
[18] Cahill MA: Progesterone Receptor Membrane Component 1: an integrative review. J Ster Biochem Mol Biol 2007;104:In Press.
[19] Runko E, Kaprielian Z: Caenorhabditis elegans VEM-1, a novel membrane protein, regulates the guidance of ventral nerve cord-associated axons. J Neurosci 13-10-2004;24:9015-9026.
[20] Anderson RG: The caveolae membrane system. Annu Rev Biochem 1998;67:199-225.
[21] Simons K, Ikonen E: Functional rafts in cell membranes. Nature 5-6-1997;387:569-572.
[22] Sbaa E, Frerart F, Feron O: The double regulation of endothelial nitric oxide synthase by caveolae and caveolin: a paradox solved through the study of angiogenesis. Trends Cardiovasc Med 2005;15:157-162.
[23] Umebayashi K: The roles of ubiquitin and lipids in protein sorting along the endocytic pathway. Cell Struct Funct 2003;28:443-453.
[24] Sun Y: E3 ubiquitin ligases as cancer targets and biomarkers. Neoplasia 2006;8:645-654.
[25] Serafini T, Kennedy TE, Galko MJ, Mirzayan C, Jessell TM, Tessier-Lavigne M: The netrins define a family of axon outgrowth-promoting proteins homologous to C. elegans UNC-6. Cell 12-8-1994;78:409-424.
[26] de Castro F: Chemotropic molecules: guides for axonal pathfinding and cell migration during CNS development. News Physiol Sci 2003;18:130-136.
[27] Keino-Masu K, Masu M, Hinck L, Leonardo ED, Chan SS, Culotti JG, Tessier-Lavigne M: Deleted in Colorectal Cancer (DCC) encodes a netrin receptor. Cell 18-10-1996;87:175-185.
[28] Shirasaki R, Mirzayan C, Tessier-Lavigne M, Murakami F: Guidance of circumferentially growing axons by netrin-dependent and -independent floor plate chemotropism in the vertebrate brain. Neuron 1996;17:1079-1088.
[29] Jia L, Emmons SW: Genes that control ray sensory neuron axon development in the Caenorhabditis elegans male. Genetics 2006;173:1241-1258.
[30] Kennedy TE: Cellular mechanisms of netrin function: long-range and short-range actions. Biochem Cell Biol 2000;78:569-575.
[31] Matsunaga E, Chedotal A: Repulsive guidance molecule/neogenin: a novel ligand-receptor system playing multiple roles in neural development. Dev Growth Differ 2004;46:481-486.
[32] Matsunaga E, Nakamura H, Chedotal A: Repulsive guidance molecule plays multiple roles in neuronal differentiation and axon guidance. J Neurosci 31-5-2006;26:6082-6088.
[33] Burgess RW, Jucius TJ, Ackerman SL: Motor axon guidance of the mammalian trochlear and phrenic nerves: dependence on the netrin receptor Unc5c and modifier loci. J Neurosci 24-5-2006;26:5756-5766.
[34] Mehlen P, Fume C: Netrin-1: when a neuronal guidance cue turns out to be a regulator of tumorigenesis. Cell Mol Life Sci 2005;62:2599-2616.
[35] Bredesen DE, Mehlen P, Rabizadeh S: Receptors that mediate cellular dependence. Cell Death Differ 2005;12:1031-1043.
[36] Arakawa H: Netrin-1 and its receptors in tumorigenesis. Nat Rev Cancer 2004;4:978-987.
[37] Xie Y, Hong Y, Ma XY, Ren XR, Ackerman S, Mei L, Xiong WC: DCC-dependent phospholipase C signaling in netrin-1-induced neurite elongation. J Biol Chem 3-2-2006;281:2605-2611.
[38] Culotti JG, Merz DC: DCC and netrins. Curr Opin Cell Biol 1998;10:609-613.
[39] Stein E, Zou Y, Poo M, Tessier-Lavigne M: Binding of DCC by netrin-1 to mediate axon guidance independent of adenosine A2B receptor activation. Science 9-3-2001;291:1976-1982.
[40] Brose K, Tessier-Lavigne M: Slit proteins: key regulators of axon guidance, axonal branching, and cell migration. Curr Opin Neurobiol 2000;10:95-102.
[41] Zhu XJ, Wang CZ, Dai PG, Xie Y, Song NN, Liu Y, Du QS, Mei L, Ding YQ, Xiong WC: Myosin X regulates netrin receptors and functions in axonal path-finding. Nat Cell Biol 2007;9:184-192.
[42] Gitai Z, Yu TW, Lundquist EA, Tessier-Lavigne M, Bargmann CI: The netrin receptor UNC-40/DCC stimulates axon attraction and outgrowth through enabled and, in parallel, Rac and UNC-115/AbLIM. Neuron 9-1-2003;37:53-65.
[43] Forcet C, Stein E, Pays L, Corset V, Llambi F, Tessier-Lavigne M, Mehlen P: Netrin-1-mediated axon outgrowth requires deleted in colorectal cancer-dependent MAPK activation. Nature 23-5-2002;417:443-447.
[44] Xie Y, Ding YQ, Hong Y, Feng Z, Navarre S, Xi CX, Zhu XJ, Wang CL, Ackerman SL, Kozlowski D, Mei L, Xiong WC: Phosphatidylinositol transfer protein-alpha in netrin-1-induced PLC signalling and neurite outgrowth. Nat Cell Biol 2005;7:1124-1132.
[45] Barallobre MJ, Pascual M, Del Rio JA, Soriano E: The Netrin family of guidance factors: emphasis on Netrin-1 signalling. Brain Res Brain Res Rev 2005;49:22-47.
[46] Bartoe JL, McKenna WL, Quan TK, Stafford BK, Moore JA, Xia J, Takamiya K, Huganir RL, Hinck L: Protein interacting with C-kinase 1/protein kinase Calphamediated endocytosis converts netrin-1-mediated repulsion to attraction. J Neurosci 22-3-2006;26:3192-3205.
[47] Galko MJ, Tessier-Lavigne M: Function of an axonal chemoattractant modulated by metalloprotease activity. Science 25-8-2000;289:1365-1367.
[48] Pasquale E: Neurobiology. Turning attraction into repulsion. Science 25-8-2000;289:1308-1310.
[49] Taniguchi Y, Kim SH, Sisodia SS: Presenilin-dependent "gamma-secretase" processing of deleted in colorectal cancer (DCC). J Biol Chem 15-8-2003;278:30425-30428.
[50] Parent AT, Barnes NY, Taniguchi Y, Thinakaran G, Sisodia SS: Presenilin attenuates receptor-mediated signaling and synaptic function. J Neurosci 9-2-2005;25:1540-1549.
[51] Viero C, Mechaly I, Aptel H, Puech S, Valmier J, Bancel F, Dayanithi G: Rapid inhibition of Ca(2+) influx by neurosteroids in murine embryonic sensory neurones. Cell Calcium 10-6-2006.
[52] Sakamoto H, Ukena K, Takemori H, Okamoto M, Kawata M, Tsutsui K: Expression and localization of 25-Dx, a membrane-associated putative progesterone-binding protein, in the developing Purkinje cell. Neuroscience 2004;126:325-334.
[53] Vetrivel KS, Cheng H, Kim SH, Chen Y, Barnes NY, Parent AT, Sisodia SS, Thinakaran G: Spatial segregation of gamma-secretase and substrates in distinct membrane domains. J Biol Chem 8-7-2005;280:25892-25900.
[54] Bouchard JF, Moore SW, Tritsch NX, Roux PP, Shekarabi M, Barker PA, Kennedy TE: Protein kinase A activation promotes plasma membrane insertion of DCC from an intracellular pool: A novel mechanism regulating commissural axon extension. J Neurosci 24-3-2004;24:3040-3050.
[55] Fume C, Corset V, Herincs Z, Cahuzac N, Hueber AO, Mehlen P: The dependence receptor DCC requires lipid raft localization for cell death signaling. Proc Natl Acad Sci U S A 14-3-2006;103:4128-4X33.
[56] Nikolopoulos SN, Giancotti FG: Netzan-integrin signaling in epithelial morphogenesis, axon guidance and vascular patterning, Cell Cycle 2005;4:e131-e135.
[57] Cole SJ, Bradford D, Cooper HM: Neogenin: A nuulti-functional receptor regulating diverse developmental processes. Int J Biochem Cell Biol 23-11-2006.
[58] Park KW, Crouse D, Lee M, Karnik SK, Sorensen LK, Murphy KJ, Kuo CJ, Li DY: The axonal attractant Netrin-1 is an angiogenic factor. Proc Natl Acad Sci U S A 16-11-2004;101:16210-16215.
[59] Nguyen A, Cai H: Netrin-1 induces angiogenesis via a DCC-dependent ERK1/2-eNOS feed-forward mechanism. Proc Natl Acad Sci U S A 25-4-2006;103:6530-6535.
[60] Hughes AL, Powell DW, Bard M, Eckstein J, Barbuch R, Link AJ, Espenshade PJ: Dapl/PGRMCl binds and regulates cytochrome P450 enzymes. Cell Metab 2407;5:143-149.
[61] Brouet A, Sonveaux P, Dessy C, Moniotte S, Balligand JL, Feron O: Hsp90 and caveolin are key targets for the proangiogenic nitric oxide-mediated effects of statins. Circ Res 9-11-2001;89:866-873.
[62] Kang JS, Yi MJ, Zhang W, Feinleib JL, Cole F, Krauss RS: Netrins and neogenin promote myotube formation. J Cell Biol 8-11-2004;167:493-504.
[63] Krauss RS, Cole F, Gaio U, Takaesu G, Zhang W, Kang JS: Close encounters: regulation of vertebrate skeletal myogenesis by cell-cell contact. J Cell Sci 1-6-2005;118:2355-2362.
[64] Bazzoni G, Dejana E: Endothelial cell-to-cell junctions: molecular organization and role in vascular homeostasis. Physiol Rev 2004;84:869-901.
[65] Martin M, Siinon-Assmann P, Kedinger M, Martin M, Mangeat P, Real FX, Fabre M: DCC regulates cell adhesion in human colon cancer derived HT-29 cells and associates with ezrin. Eur J Cell Biol 2006;85:769-783.
[66] Zhang AS, West AP, Jr., Wyman AE, Bjorkman PJ, Enns CA: Interaction of hemojuvelin with neogenin results in iron accumulation in human embryonic kidney 293 cells. J Biol Chem 7-10-2005;280:33885-33894.
[67] Valko M, Rhodes CJ, Moncol J, Izakovic M, Mazur M: Free radicals, metals and antioxidants in oxidative stress-induced cancer. Chem Biol Interact 10-3-2006;160:1-40.
[68] Slorach EM, Werb Z: Epithelial morphogenesis: Netrin comes to a sticky and terminal end. Curr Biol 17-6-2003;13:R491-R493.
[69] Hinck L, Silberstein GB: Key stages in mammary gland development: the mammary end bud as a motile organ. Breast Cancer Res 2005;7:245-251.
[70] Strizzi L, Bianco C, Raafat A, Abdallah W, Chang C, Raafat D, Hirota M, Hamada S, Sun Y, Normanno N, Callahan R, Hinck L, Salomon D: Netrin-1 regulates invasion and migration of mouse mammary epithelial cells overexpressing Cripto-1 in vitro and in vivo. J Cell Sci 15-10-2005;118:4633-4643.
[71] Huber MA, Kraut N, Beug H: Molecular requirements for epithelial-mesenchymal transition during tumor progression. Curr Opin Cell Biol 2005;17:548-558.
[72] Yang J, Mani SA, Donaher JL, Ramaswamy S, Itzykson RA, Come C, Savagner P, Gitelman I, Richardson A, Weinberg RA: Twist, a master regulator of morphogenesis, plays an essential role in tumor metastasis. Cell 25-6-2004;117:927-939.
[73] Kang Y, Massague J: Epithelial-mesenchymal transitions: twist in development and metastasis. Cell 6-8-2004;118:277-279.
[74] Vernon AE, LaBonne C: Tumor metastasis: a new twist on epithelial-mesenchymal transitions. Curr Biol 7-9-2004;14:R719-R721.
[75] van', V, Dai H, van d, V, He YD, Hart AA, Mao M, Peterse HL, van der KK, Marton MJ, Witteveen AT, Schreiber GJ, Kerkhoven RM, Roberts C, Linsley PS, Bernards R, Friend SH: Gene expression profiling predicts clinical outcome of breast cancer. Nature 31-1-2002;415:530-536.
[76] Lee JE, Kim HJ, Bae JY, Kim SW, Park JS, Shin HJ, Han W, Kim SW, Kang KS, Noh DY: Neogenin expression may be inversely correlated to the tumorigenicity of human breast cancer. BMC Cancer 2005;5:154.
[77] Velardo MJ, Burger C, Williams PR, Baker HV, Lopez MC, Mareci TH, White TE, Muzyczka N, Reier PJ: Patterns of gene expression reveal a temporally orchestrated wound healing response in the injured spinal cord. J Neurosci 29-9-2004;24:8562-8576.
[78] Heaton JH, Dlakic WM, Dlakic M, Gelehrter TD: Identification and cDNA cloning of a novel RNA-binding protein that interacts with the cyclic nucleotide-responsive sequence in the Type-1 plasminogen activator inhibitor mRNA. J Biol Chem 2-2-2001;276:3341-3347.
[79] Song HJ, Poo MM: Signal transduction underlying growth cone guidance by diffusible factors. Curr Opin Neurobiol 1999;9:355-363.
[80] Corset V, Nguyen-Ba-Charvet KT, Forcet C, Moyse E, Chedotal A, Mehlen P: Netrin-1-mediated axon outgrowth and cAMP production requires interaction with adenosine A2b receptor. Nature 12-10-2000;407:747-750.
[81] Ming G, Henley J, Tessier-Lavigne M, Song H, Poo M: Electrical activity modulates growth cone guidance by diffusible factors. Neuron 2001;29:441-452.
[82] Shewan D, Dwivedy A, Anderson R, Holt CE: Age-related changes underlie switch in netrin-1 responsiveness as growth cones advance along visual pathway. Nat Neurosci 2002;5:955-962.
[83] Nishiyama M, Hoshino A, Tsai L, Henley JR, Goshima Y, Tessier-Lavigne M, Poo MM, Hong K: Cyclic AMP/GMP-dependent modulation of Ca2+ channels sets the polarity of nerve growth-cone turning. Nature 26-6-2003;423:990-995.
[84] Hand RA, Craven RJ: Hpr6.6 protein mediates cell death from oxidative damage in MCF-7 human breast cancer cells. J Cell Biochem 15-10-2003;90:534-547.
[85] Swanson KM, Hohl RJ: Anti-cancer therapy: targeting the mevalonate pathway. Curr Cancer Drug Targets 2006;6:15-37.
[86] Crudden G, Chitti RE, Craven RJ: Hpr6 (heme-1 domain protein) regulates the susceptibility of cancer cells to chemotherapeutic drugs. J Pharmacol Exp Ther 2006;316:448-455.
[87] Dimitroulakos J, Nohynek D, Backway KL, Hedley DW, Yeger H, Freedman MH, Minden MD, Penn LZ: Increased sensitivity of acute myeloid leukemias to lovastatin-induced apoptosis: A potential therapeutic approach. Blood 15-2-1999;93:1308-1318.

## Claims

1. Use of at least one isoform of progesterone receptor membrane component 1 (PGRMC1) as diagnostic marker and/or therapeutic target for diseases associated with neogenin and/or DCC (Deleted in colorectal cancer).

2. Use of at least one isoform of progesterone receptor membrane component 1 (PGRMC1) for the manufacture of a medicament for treatment of diseases associated with neogenin and/or DCC.

3. Use of at least one reagent that influences the function of at least one isoform of progesterone receptor membrane component 1 (PGRMC1) for the manufacture of a medicament for treatment of diseases associated with neogenin and/or DCC.

4. Use according to claim 1 or 2, **characterized in that** at least two or three isoforms are used as diagnostic marker and/or therapeutic target or in the manufacture of the medicament.

5. Use according to one of the preceding claims, **characterized in that** said diseases comprise neuronal disorders, preferably memory disorders, in particular in mammalians.

6. Use according to one of claims 1 to 4, **characterized in that** said diseases comprise cancer of the nervous system.

7. Use according to one of claims 1 to 4, **characterized in that** said diseases comprise cancer, in particular breast cancer.

8. Use according to one of claims 1 to 4, **characterized in that** said diseases comprise inflammatory diseases, in particular atherosclerosis or rheumatoid arthritis.

9. Use according to one of claims 1 to 4, **characterized in that** said diseases comprise cardiovascular diseases.

10. Use according to one of claims 6 or 7, **characterized in that** at least one of said isoforms of PGRMC1 has a significantly increased abundance in cancer cells of said cancers.

11. Use according to one of the preceding claims, **characterized in that** said isoforms of PGRMC1 differ from each other in their phosphorylation status.

12. Use according to claim 10, **characterized in that** said cancer cells are negative for the estrogen receptor (ER-).

13. Use according to claim 10, **characterized in that** said cancer cells are negative for the progesterone receptor (PR-).

14. Use of at least one isoform of progesterone receptor membrane component 1 (PGRMC1) for influencing, in particular increasing, abundance or subcellular localisation of at least one class of molecules interacting with PGRMC1.

15. Use of at least one isoform of progesterone receptor membrane component 1 (PGRMC1) for influencing, in particular decreasing, abundance or subcellular localisation of at least one class of molecules interacting with PGRMC1.

16. Use according to claim 14 or 15, **characterized in that** said class of molecule is at least one of Deleted in colorectal cancer (DCC) or Neogenin.

17. Use according to claim 14 or 15, **characterized in that** said class of molecule is a ubiquitin ligase.

18. Use according to claim 14 or 15, **characterized in that** said class of molecule is a polyubiquitinase.

19. Use according to claim 3, **characterized in that** said at least one reagent increases or decreases the function or changes the subcellular locality of of PGRMC1.

20. Use according to claim 3, **characterized in that** said at least one reagent is a reagent for influencing, in particular increasing or decreasing, the phosphorylation status of the at least one isoform of PGRMC1.

21. Use according to claim 3, **characterized in that** said reagent is an antibody or small molecule affinity reagent directed against PGRMC1.

22. Assay kit for diagnosis and/or therapy of diseases associated with neogenin and/or DCC, comprising at least one isoform of progesterone receptor membrane component 1 (PGRMC1).

23. Assay kit for diagnosis and/or therapy of diseases associated with neogenin and/or DCC, comprising at least one reagent that influences the function of at least one isoform of progesterone receptor membrane component 1 (PGRMC1).

24. Use of at least one isoform of progesterone receptor membrane component 1 (PGRMC1) as diagnostic marker in diagnosis for diseases associated with aberrant biological phenotypes, wherein the phosphorylation status of the at least one isoform of progesterone receptor membrane component 1 (PGRMC1) is determined/and or estimated.

25. Use according to claim 24, **characterized in that** the phosphorylation status is determined and/or estimated by analysis of proteins that are involved, in particular differentially involved, in protein interaction or multi-protein complexes with either phosphorylated or non-phosphorylated at least one isoform of progesterone receptor membrane component 1 (PGRMC1).

26. Use of at least one reagent for influencing, in particular increasing or inhibiting, the abundance and/or activity of isoforms of proteins for diagnosis and/or therapy of diseases associated with aberrant biological phenotypes, wherein said proteins are involved, in particular differentially involved, in protein interaction or multi-protein complexes with either at least one isoform of phosphorylated or non-phosphorylated progesterone receptor membrane component 1 (PGRMC1).

27. Use according to claim 26, **characterized in that** said diseases, in particular subgroups thereof, comprise cancer, especially breast cancer or prostate cancer, neurodegenerative diseases, infertility, inflammatory, immunological, respiratory, pulmonary diseases, and/or diseases associated with the rate of biological aging or with beneficial or detrimental alterations of the level of the process of autophagy.
